# EUROPEAN PATENT SPECIFICATION

(11) **EP 3 788 999 B1**
(45) Date of publication and mention of the grant of the patent: **07.06.2023**
(21) Application number: 20196693.4
(22) Date of filing: 29.03.2018
(51) Int. Cl.: A61F 13/476, A61F 13/551, A61F 13/56, A61F 13/15

(54) **FEMININE ABSORBENT ARTICLES WITH BONDED SIDE FLAPS AND AN APPARATUS FOR PRODUCING THE SAME**
SAUGFÄHIGE ARTIKEL FÜR DAMEN MIT GEBUNDENEN SEITENLASCHEN UND VORRICHTUNG ZUR HERSTELLUNG DAVON
ARTICLES ABSORBANTS FÉMININS DOTÉS DE RABATS LATÉRAUX LIÉS ET APPAREIL DE PRODUCTION ASSOCIÉ

(30) Priority: 31.03.2017 US 201762479812 P
(43) Date of publication of application: 10.03.2021
(62) Divisional of application: 18725943.7
(73) Proprietor: Johnson & Johnson Consumer Inc., Skillman, NJ 08558 (US)
(72) Inventor: CAU, Jose Francisco, 12241-070 São José Dos Campos/SP (BR); ALKMIN, Marco Antonio, 12241-070 São José Dos Campos/SP (BR); SARDINHA, Gilson Philigret, 12241-070 São José Dos Campos/SP (BR)
(74) Representative: Carpmaels & Ransford LLP

(56) References cited:
- EP-A2- 1 142 546
- WO-A1-98/53781
- US-A- 5 800 654
- US-A1- 2002 156 448
- US-A1- 2005 124 958
- US-A1- 2015 351 978

## Description

### FIELD OF THE INVENTION

The present invention relates to feminine absorbent articles with bonded side flaps. More specifically, the invention relates to a method of making feminine absorbent articles with bridging side flaps.

### BACKGROUND OF THE INVENTION

Sanitary napkins are low cost mass produced articles. A typical manufacturing facility includes an assembly line where the various components of the sanitary napkin are progressively combined and laminated into a continuous web, which is cut transversely into discrete products.

The term "sanitary napkin", as used herein, refers to an article that is worn by females in their undergarments adjacent to the pudendal region and which is intended to absorb and contain the various exudates that are discharged from the body (e.g., blood, menses, vaginal discharges and urine). Hence, the term "sanitary napkin" encompasses pantiliners as well as catamenial devices. The term "disposable" refers to articles that are intended to be discarded after a single use. That is, the articles are not intended to be laundered or otherwise restored or reused as an absorbent article.

To enhance the functionality of sanitary napkins, manufacturers are designing products with complex and sophisticated contour lines. For example, it is known to provide sanitary napkins with flaps having adhesive disposed thereon, the flaps being adapted to be folded over and secured to the undergarment of the user via the adhesive, thereby enabling the secure attachment of the sanitary napkin to the undergarment. With traditional manufacturing techniques, extensive trimming is required to produce the highly irregular contoured edge of such sanitary napkins, which generates a considerable amount of waste material. As a result, the manufacturing cost of the sanitary napkin increases because the starting material is used less efficiently.

The industry has developed sanitary napkins with one or more flaps which can be produced with a reduced amount of waste material as compared to prior art sanitary napkins. Methods of manufacturing flapped absorbent articles where the flaps are bonded instead of making them from an extension of the product impermeable barrier and/or the cover layers are known to provide the advantage of reducing the amount of "scrap" in the end of the manufacturing process.

Such processes are also known to allow the production of flapped absorbent articles where the flaps are made from materials presenting properties that are not found in other product layers, and hence achieving a specific elasticity, air permeability or any other desired mechanical property which could not be achieved by simply making the flaps from an extension from the product impermeable barrier and/or the cover layers.

However, with the advantages in bonding the referred flaps comes increases in the complexity of manufacturing methods and apparatus. So, there is a need for a method and apparatus for manufacturing feminine absorbent articles including bonded flaps that is reduced in complexity.

Additionally, such processes are also known to be limited to produce absorbent articles having their longitudinal axis aligned with the machine's manufacturing direction, where such configuration is known to provide a reduced rate of production when compared to similar machines that manufactures the feminine absorbent articles having their longitudinal axis transversally oriented to the manufacturing direction. So, there is a need for a method and apparatus for manufacturing feminine absorbent articles including bonded flaps that manufactures those articles transversely oriented to the manufacturing direction.

Furthermore, there is a need for a method and apparatus for manufacturing feminine absorbent articles including bonded flaps that is reduced in complexity and that manufactures those articles transversely oriented to the manufacturing direction.

US20020156448A1 discloses an absorbent article, preferably a sanitary napkin, having a body-facing side, a garment-facing side, a length, a width, and two longitudinal side margins. The sanitary napkin comprises a liquid pervious topsheet, a liquid impervious backsheet joined to said topsheet, and an absorbent core positioned between the topsheet and said backsheet. A pair of flaps are provided, each flap extending from a longitudinal side margin and each of the flaps are folded over the topsheet in a topsheet facing relationship.

WO1998053781A1 discloses an absorbent article that extends in a longitudinal direction and comprises a main body portion having a pair of longitudinal side edges, a pair of end edges, a garment surface, and a body surface. The absorbent article further comprises a pair of first flaps joined to the main body portion and extending laterally outward beyond the longitudinal side edges of the main body portion, and a pair of second flaps joined to the main body portion apart from the first flaps in the longitudinal direction and extending laterally outward beyond the longitudinal side edges of the main body portion. The garment surface of each of the first and second flaps comprises a first flap fastener and a second flap fastener respectively. The first and second flaps are folded over the body surface of the main body portion to expose the flap fasteners.

US20150351978A1 discloses absorbent articles that have a chassis with a topsheet, a backsheet, and an absorbent core disposed therebetween. A pair of side flaps is attached to the chassis. A portion of each first surface of the pair of side flaps is attached to an outer-facing surface of the chassis. Another portion of each first surface has adhesive disposed thereon. A release liner covers at least a portion of the adhesive of the first surface. The release liner and free ends of each of the pair of side flaps are superjacent to the chassis prior to use.

### SUMMARY OF THE INVENTION

We have found novel methods for the improved manufacture of sanitary napkins having side flaps or wings.

In one embodiment, a method for manufacturing disposable sanitary napkins intended to be worn in an undergarment of a user includes the steps of forming pairs of bridged flaps, attaching the bridged flaps to a main body of the sanitary napkin, and enclosing the sanitary napkin in packaging material. The bridged flaps are formed by providing a continuous web having a longitudinal axis and at least a garment-facing layer and an opposite layer, bonding said superposed layers to form an area of juncture on said continuous web, said area of juncture defining a cyclic pattern and extending along said longitudinal axis, severing said continuous web within said area of juncture to form first and second shaped strips, adjusting the two shaped strips to align the projecting portions of the first shaped strip adjacent the projecting portions of the second shaped strip, whereby the adjusted shaped strips have outer edges defined by the first, substantially straight edges of the first and second shaped strips, applying zones of pressure sensitive adhesive to a continuous web of release liner, each zone of pressure sensitive adhesive corresponding to a projecting portion of the first and second shaped strips, and severing bridging segments of the release liner, each bridging segment having at least one zone of pressure sensitive adhesive arranged and configured for attachment to a projecting portion of the first shaped strip and at least one zone of pressure sensitive adhesive arranged and configured for attachment to a projecting portion of the second shaped strip, adjacent the projecting portion of the first shaped strip. Each strip has a first, substantially straight edge, a second, shape-cut edge defining a plurality of projecting portions in a spaced apart relationship, and at least one attachment portion disposed along the first edge between each of said projecting portions that unite said projecting portions to one another. The bridged flaps are attached to the main body in a manner wherein each shaped strip is bonded to the topsheet such that the first, substantially straight edge of each shaped strip is aligned with a corresponding side edge of the main body, which corresponds to a side edge of the topsheet, the bridged flaps are disposed over the topsheet, and each side flap is arranged and configured to be releasable from the bridging segment of the release liner and to be articulable away from the topsheet of the sanitary napkin and to be secured to a crotch portion of an undergarment for use. The main body of the sanitary napkin has a length, a width, and a thickness, includes an absorbent system, a topsheet, and a backsheet, and has a pair of side edges extending along the length thereof.

In one preferred embodiment, the bridged flaps are attached to the main body as the main body travels in a direction perpendicular to its length transverse to the disposable sanitary napkin.

In another embodiment, a disposable sanitary napkin intended to be worn in an undergarment of a user includes a main body and a pair of bridged flaps. The main body has a length, a width, and a thickness, and it includes an absorbent system, a topsheet, and a backsheet. The main body also has a pair of side edges extending along the length thereof. The bridged flaps include a first, substantially straight edge, a second, shape-cut edge defining a projecting portion, and a bridging segment of a release liner having at least one zone of pressure sensitive adhesive arranged and configured for attachment to the projecting portion of each bridged flap. Each bridged flap is bonded to the topsheet such that the first, substantially straight edge of each shaped strip is aligned with a corresponding side edge of the main body, wherein the corresponding side edge of the main body is a corresponding side edge of the topsheet, the bridged flaps are disposed over the topsheet, and each side flap is arranged and configured to be releasable from the bridging segment of the release liner and to be articulable away from the topsheet of the sanitary napkin and to be secured to a crotch portion of an undergarment for use.

### BRIEF DESCRIPTION OF THE DRAWINGS

FIG. 1 is a perspective view of a feminine absorbent article made according to the present invention;
FIG. 2 is a cross-sectional view of the absorbent article of FIG. 1 along the II-II' plain;
FIG. 3 is a descriptive schematic of the apparatus of the present invention;
FIG. 4 is a top view of a pair of strips as they transition from the wing synchronizer station to the release liner cut and place station;
FIG. 5 is a schematic of the release liner cut and place station;
FIG. 6 is a top view of strip of bridged flaps as they transition from the release liner cut and place station to the flap kit maker;
FIG. 7 is a schematic of the flap kit maker;
FIG. 8 is a top view of unitary flap kit;
FIG. 9 is a bottom view of unitary flap kit upon exit from the flap kit maker; and
FIG. 10 is a schematic of the rotary wheel.

### DETAILED DESCRIPTION OF THE INVENTION

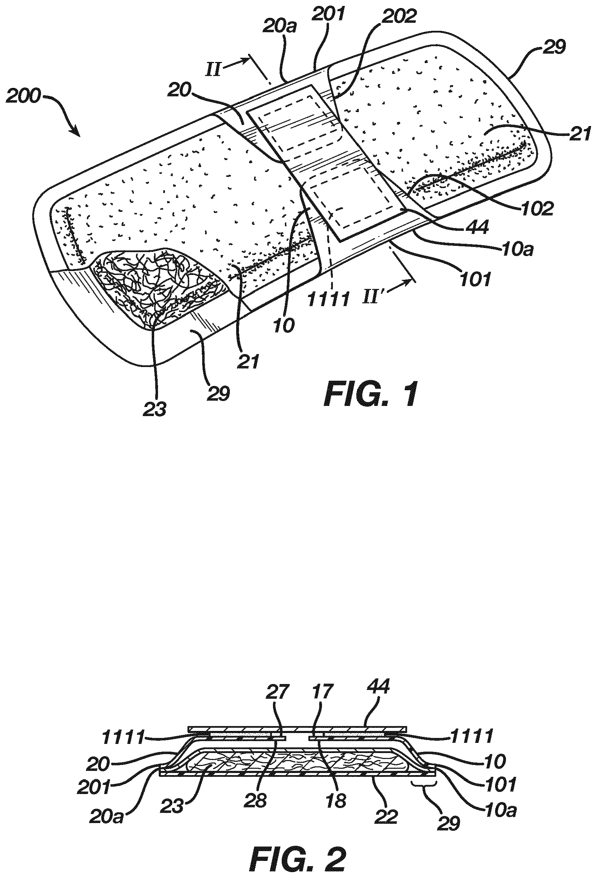

FIGs. 1 and 2 show and embodiment of a feminine absorbent article **200** made according to the present invention. FIG. 1 is a perspective view of the absorbent article **200,** while FIG. 2 is a cross-sectional view of the absorbent article of FIG. 1 along the II-II' plain. The feminine absorbent article **200** has a liquid-permeable topsheet **21,** a liquid-impermeable backsheet **22** bonded to the topsheet **21,** a liquid-absorbent core **23** disposed between the topsheet **21** and the backsheet **22.** Feminine absorbent article **200** further includes a peripheral region **29** where the topsheet **21** and the backsheet **22** are bonded together.

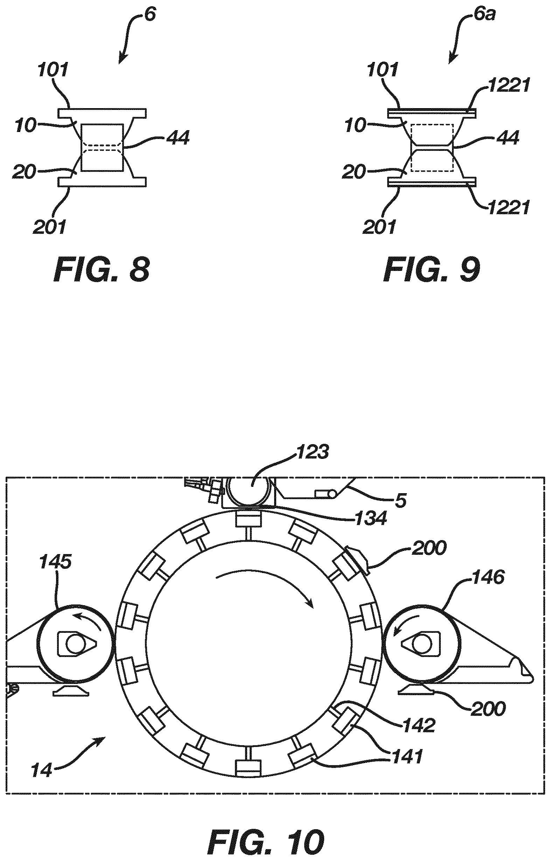

Article **200** further includes a first side flap **10** and a second side flap **20.** First side flap **10** and a second side flap **20** may also be referred to as first wing **10** and second wing **20.** First side flap **10** has a first surface **17** and a second surface **18,** as well as straight lateral edge 101 and shape-cut edge **102.** Second side flap **20** has a first surface **27** and a second surface **28,** as well as straight edge **201** and shape-cut edge **202.** First side flap **10** is bonded to topsheet **21** along a region of first surface **17** adjacent to its straight lateral edge 101 in peripheral region **29.** Likewise, second side flap **20** is bonded to topsheet **21** along a region of first surface **27** adjacent to its straight edge **201** in peripheral region **29.** The bonding means for bonding first side flap **10** and second side flap **20** to peripheral region **29** may include an adhesive weld, a heat weld, or an ultrasonic weld. The bonding of the first side flap **10** to the peripheral region **29** and the second side flap **20** to the peripheral region **29** forms flanges **10a, 20a** adjacent the straight lateral edges **101, 201.** In one embodiment, shown in FIG. 9, the bonding means is a layer of adhesive **1221.**

Second surface **18** of first side flap **10** includes a region with fastening adhesive **1111,** as does second surface **28** of second side flap **20.** First side flap **10** and second side flap **20** are folded over topsheet **21,** and are releasable bonded by a discrete piece of a release liner **44** placed on each layer of fastening adhesive **1111.** The fastening adhesive may be any adhesive known to those of ordinary skill in the art, including without limitation, hot melt adhesives and solventbased adhesives.

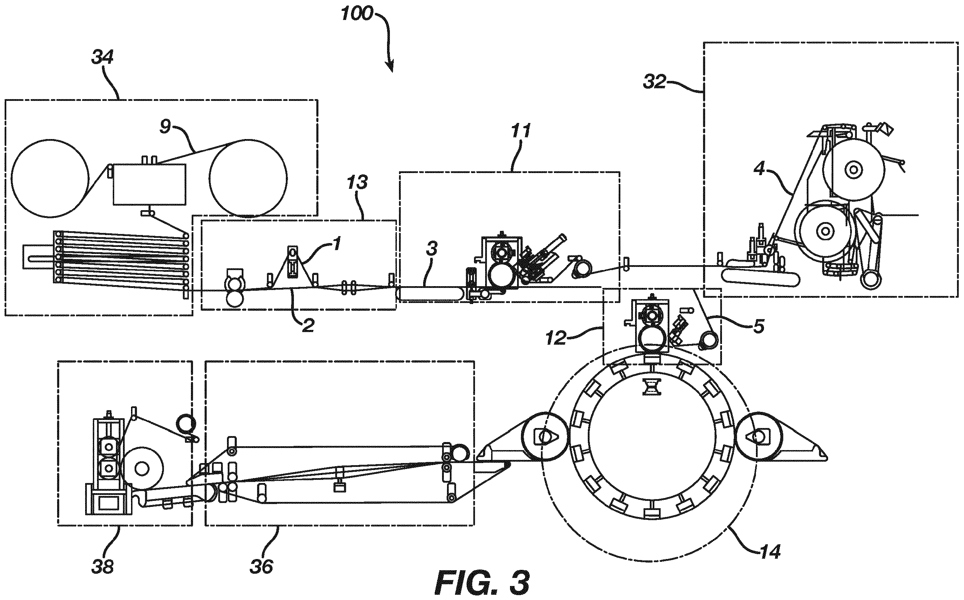

FIG. 3 is a descriptive schematic of the apparatus **100** of the present invention. Apparatus **100** includes a release paper unwinder system **32,** a wing material unwinder system **34,** a wing synchronizer station **13,** a release liner cut and place station **11,** a flap kit maker **12,** a rotary wheel **14,** a twister **36,** and a die cutter **38.**

The release paper unwinder system **32** is aimed to properly feed the apparatus with the release liner **4** to the apparatus and further obtaining discrete pieces of release liner **44.** It typically comprises a set of unwinder rolls and tensioning means.

The set of unwinder rolls uses two servo driven shafts and gripping means to engage the shaft inside the roll of release lines 4 (as a rolled starting material). The gripping means may consist of mechanic of pneumatic elements on the shaft.

The speed of the unwinding rolls is preferentially controlled by a PLC which manages their rotation speed by instantaneously reading the roll diameter.

An automatic splice unit works in synchronism with the unwinders allowing a perfect splicing from one roll to the next one at production speed and with the smallest waste of raw material. This unit uses pneumatic cylinders, vacuum conveyor, knives and pressing rolls. The automatic splicing starts by a signal from the roll sensors to the PLC informing that the minimum diameter has been achieved by the roll which is in use. In this moment, the vacuum conveyor receives a message from the PLC to starts feeding the raw material from the new roll. At the same time the roll which is in use stops.

The tensioning means may consist of a set of rolls or a vacuum featured conveyor. Preferentially it will be selected from a vacuum conveyor system, taking in consideration the mechanical properties of the release paper **4.**

The wing material unwinder system **34** is aimed to properly feed the apparatus with the single strip **9** that will further be turned in side flaps **10/20.** Similarly to the release paper unwinder system **32,** the wing material unwinder system **34** typically comprises a set of unwinder rolls and tensioning means. Due to the mechanical properties of the single strip **9,** the selected tension means may include a festoon, which will store some release liner **9** that will be consumed during the change of unwinder rolls.

The wing synchronizer station **13** is used as a means for managing the substrate strip **9** used to form first wing **10** and second wing **20.** Suitable means for managing substrate strip **9** are known in the art; typically, they consist of an arrangement, a station, capable of splitting a single substrate strip **9** in two strips. The strips have a non-linear cut, providing shaped substrate strips having a straight lateral edge **101** and a shape-cut edge **102** which are used to construct side flaps **10** and **20.**

An exemplary arrangement for manufacturing shaped strips is described in the published U.S. Pat. US20050124958.

Typically, wing synchronizer station **13** includes cutting means like knives to split single substrate strip **9** into a first strip **1** and a second strip **2.** Wing synchronizer station **13** also includes a series of spacer elements to move longitudinal and transversally first strip **1** in relation to second strip **2** to assisting the desired placement of the strips onto the products, in a further step of the process.

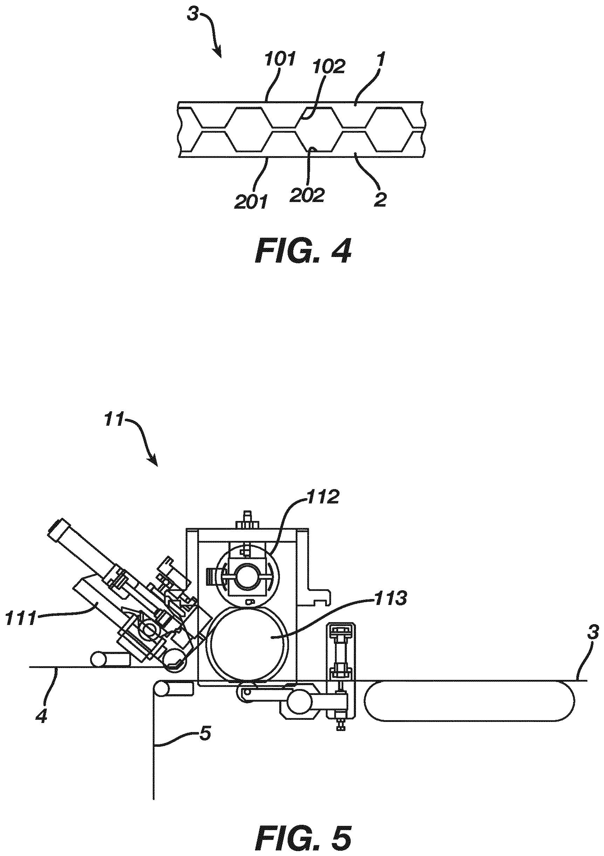

FIG. 4 is a top view of a pair of strips **3** as they transition from the wing synchronizer station **13** to the release liner cut and place station **11.** Wing synchronizer station **13** will result in first strip **1** and second strip **2.** First strip **1** has a substantially straight lateral edge **101** and a shape-cut edge **102,** as well as first surface **17** and a second surface **18.** Second strip **2** has a substantially straight lateral edge **201** and a shape-cut edge **202,** as well as first surface **27** and a second surface **28.** Shape-cut edge **102** and shape-cut edge **202** are symmetrically oriented along an imaginary longitudinal line.

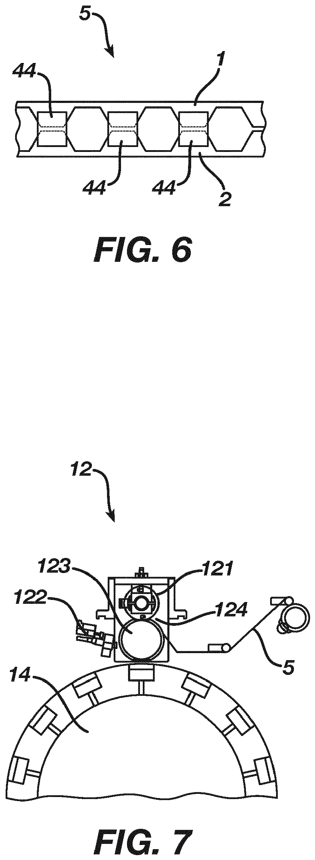

FIG. 5 is a schematic of the release liner cut and place station **11.** Release liner cut and place station **11** is fed with a pair of strips **3** obtained from the wing synchronizer station **13** and a continuous strip of release liner **4** obtained from release paper unwinder system **32.** Release liner cut and place station **11** converts pair of strips **3** and continuous strip of release liner **4** obtained into a unitary strip **5** as shown in FIG. 6.

Release liner cut and place station **11** includes a hot melt adhesive applicator **111,** knife **112,** and vacuum featured roll **113.**

Hot melt adhesive applicator **111** is used to apply hot melt adhesive **1111** onto continuous release liner strip **4.** There are several suitable adhesive applicators to the present invention, from contactless applicators to contact applicators. Contactless applicators which may be used in some embodiments, are those which sprays the adhesive, such those found in the PATERN JET spray applicators, or SPEED-COAT slot applicators, supplied from Nordson Corporation (Westlake, OH). Contact applicators, which may be used in some embodiments, are like those found in the TRUECOAT line of applicators, supplied from Nordson Corporation (Westlake, OH).

Knife **112** used in release liner cut and place station **11,** is preferentially a roll kind of knife that cooperates with vacuum featured roll **113** to create a cutting nip where the release liner **4** will be cut and converted into discrete pieces of release liner **44.** Once leaving the cutting nip, discrete pieces of release liner **44** are conveyed to pair of strips **3** by vacuum featured roll **113.** Once the discrete pieces of release liner **44** leave vacuum featured roll **113,** they will remain releasable bonded to pair of strips **3** by the hot melt adhesive **1111.** As a result, pair of strips **3** shown in FIG. 3 are converted into a unitary strip **5** shown in FIG. 6. Preferentially, some pressure will be performed by vacuum featured roll **113** when transferring the discrete pieces of release liner **44** to pair of strips **3,** in order to achieve bonding strength between them.

Release liner cut and place station **11** is further capable of selectively separating one discrete piece of release liner **44** from another, in order to place them onto pair of strips **3** in such way to bridge pair of strips **3** by means of hot melt adhesive **1111.** Separation and bridging is accomplished by managing the tangential speed of vacuum featured roll **113,** where typically a servo-mechanical system is capable of controlling such tangential speed.

It is important to note that discrete pieces of release liner **44** are moved from the cutting nip to pair of strips **3** by the vacuum featured roll **113**because of how its vacuum chambers are configured. A specialist in such kind of machines will be able to properly decide where to place the vacuum chambers, in order to properly convey discrete pieces of release liner **44,** until placing them onto pair of strips **3.** FIG. 6 shows a unitary strip **5** of bridged flaps as they transition from release liner cut and place station **11** to the flap kit maker **12.** The figure shows a unitary strip **5** of bridged flaps connected by discrete pieces of release liner **44.**

FIG. 7 is a schematic of the flap kit maker **12.** Flap kit maker **12** converts continuous strip **5** of bridged flaps into discrete unitary bridged flap kits **6,** with each bridged flap kit **6** consisting of a pair of side flaps (first side flap **10** and second side flap **20**) releasable bonded by a single discrete piece of release liner **44** by pressure sensitive hot melt adhesive **1111.** FIG. 8 is a top view of a unitary bridged flap kit **6** upon exit from flap kit maker **12.** Unitary bridged flap kit **6** has first side flap **10** and second side flap **20** bridged and connected by discrete piece of release liner **44.**

Flap kit maker **12** included a knife **121** and a vacuum featured wheel **123.** Knife **121,** preferentially a roll kind of knife, cooperates with vacuum featured wheel **123** to create a nip **124** where unitary strip **5** of bridged flaps will be cut and converted into separate unitary bridged flap kit **6.**

In the embodiment shown, flap kit maker **12** includes a hot melt adhesive applicator **122** aimed to apply hot melt adhesive **1221** onto second surface **18** of first side flap **10** adjacent to straight lateral edge 101 and onto second surface **28** of second side flap **20** adjacent to straight edge **201.**

FIG. 9 is a bottom view of unitary flap kit **6a** upon exit from flap kit maker **12.** The figure shows the location of hot melt adhesive **1221** on second surface **18** of first side flap **10** and second surface **28** of second side flap **20.**

FIG. 10 is a schematic of rotary wheel **14** for use in the present invention. Rotary wheel **14** cooperates with flap kit maker **12** to transfer and bond unitary flap kits **6a** onto the absorbent articles. In this sense, rotary wheel **14** and vacuum featured wheel **123** of flap kit maker **12** cooperate creating a nip **134** which selectively compress a unitary flap kit **6a** against an absorbent article; both rotary wheel **14** and the vacuum featured wheel **123** include vacuum chambers selectively arranged to promote the transfer of each unitary flap kit **6a** to a respective absorbent article. In particular product embodiments, the rotary wheel may apply more than a single unitary flap kit **6a** per product, which is typically found on overnight products.

In embodiments where unitary flap kit **6a** includes hot melt adhesive **1221** applied by adhesive applicator **122** of flap kit maker **12** on second surface **18** of first side flap **10** adjacent to straight lateral edge 101 and on second surface **28** of second side flap **20** adjacent to straight edge **201,** it will permanently bond unitary flap kit **6a** to the peripheral region **29** of feminine absorbent article **200** once article **200** has left nip **134.**

Depending on the orientation of the manufactured absorbent articles **200** in relationship to the machine's manufacturing direction, the longitudinal axis of flap kit **6a** might not be aligned with the longitudinal axis of absorbent articles **200.** In such cases, rotary wheel **14** can rotate absorbent articles **200** before they reach nip **134,** if featured with vacuum featured shoe **141.**

One exemplary rotation mean is a geared vacuum featured shoe **141.** As seen in FIG. 10, geared vacuum featured shoe **141**is connected to a vacuum source by a pneumatic connection **142.** Thus, a geared vacuum featured shoe **141** is capable of sticking to absorbent articles **200** and rotating them.

Preferentially, the absorbent articles will be conveyed from a feeding tray **145** to rotary wheel **14** and from rotary wheel **14** to collecting tray **146** by means of vacuum forces.

The twister **36** has two flat conveyors which are independently servo driven and have the function of receiving absorbent article **200** from the conveyors after the final cut and turn it 180 degrees from the original position making the bottom surface of the product assumes the top position (absorbent article **200** is turned upside down). After being turned, absorbent article **200** is delivered to the conveyor belt (feeding tray **145**) and then to the rotary wheel **14.**

The die cutter **38** does the final cut of the product to the final dimensions and configuration (product contour). This operation, final cut, is done using a pair of rotary tools (knife and anvil) which runs together and cut absorbent article **200** by squeezing. The cutting force is applied to the tool by pneumatic, hydraulic or mechanical actuators. The design waste is removed from this station by pneumatic transportation. The pair, knife and anvil, is done of hard materials and can be submitted to an operation of re-sharpening in order to recover its ability of cutting smoothly.

The disposable sanitary napkins intended to be worn in an undergarment of a user may be manufactured by forming pairs of bridged flaps, attaching the bridged flaps to the main body of the sanitary napkin, and enclosing the sanitary napkin in a packaging material. The main body has a length, a width, and a thickness, and it includes an absorbent system disposed between a topsheet, and a backsheet. The main body has a pair of side edges extending along the length thereof. The bridged flaps are formed from a continuous web having a longitudinal axis and at least a garment-facing layer and an opposite layer. These two layers are superposed to form an area of juncture on said continuous web, said area of juncture defining a cyclic pattern and extending along said longitudinal axis. The continuous web is severed within said area of juncture to form first and second shaped strips. Each shaped strip has a first, substantially straight edge, a second, shape-cut edge defining a plurality of projecting portions in a spaced apart relationship, and at least one attachment portion disposed along the first edge between each of said projecting portions that unite said projecting portions to one another.

The two shaped strips are adjusted to align the projecting portions of the first shaped strip adjacent the projecting portions of the second shaped strip. Thus, the adjusted shaped strips have outer edges defined by the first, substantially straight edges of the first and second shaped strips. Zone of pressure sensitive adhesive are applied to a continuous web of release liner, each zone of pressure sensitive adhesive corresponding to a projecting portion of the first and second shaped strips. The resulting structure is severed to provide bridging segments; having at least one zone of pressure sensitive adhesive arranged and configured for attachment to a projecting portion of the first shaped strip and at least one zone of pressure sensitive adhesive arranged and configured for attachment to a projecting portion of the second shaped strip, adjacent the projecting portion of the first shaped strip.

The bridging segment is attached to the main body as follows: the first, substantia2lly straight edge of each shaped strip is aligned with a corresponding side edge of the main body and affixed thereto with a flange seal **10a, 20a,** the bridged flaps are disposed over the topsheet, and each side flap is arranged and configured to be releasable from the bridging segment of the release liner. The side flaps are also arranged and configured to be articulable away from the topsheet of the sanitary napkin and to be secured to a crotch portion of an undergarment for use whereby the flange seals **10a, 20a** are disposed inwardly toward the crotch portion of the undergarment.

## Claims

1. A disposable sanitary napkin (200) intended to be worn in an undergarment of a user, the sanitary napkin (200) comprising:
(a) a main body:
(i) having a length, a width, and a thickness;
(ii) comprising an absorbent system (23), a topsheet (21), and a backsheet (22); and
(iii) having a pair of side edges extending along the length thereof;
(b) a pair of bridged flaps (10, 20) comprising
(i) a first, substantially straight edge (101, 201);
(ii) a second, shape-cut edge (102, 202) defining a projecting portion;
(iii) a bridging segment of a release liner having at least one zone of pressure sensitive adhesive arranged and configured for attachment to the projecting portion of each bridged flap;
wherein each bridged flap (10, 20) is attached to the top sheet such that the first, substantially straight edge (101, 201) of each shaped strip is aligned with a corresponding side edge (29) of the main body to form an outwardly disposed flange seal, the bridged flaps (10, 20) are disposed over the topsheet (21), and each side flap is arranged and configured to be releasable from the bridging segment of the release liner and to be articulable away from the topsheet (21) of the sanitary napkin and to be secured to a crotch portion of an undergarment for use whereby the flange seals are disposed inwardly toward the crotch portion of the undergarment,
wherein each bridged flap (10, 20) is bonded to the topsheet (21), and wherein the corresponding side edge of the main body is a corresponding side edge of the topsheet (21).

2. The disposable sanitary napkin (200) of claim 1, wherein each bridged flap (10, 20) is bonded to the topsheet (21) in a peripheral region wherein the topsheet (21) and backsheet (22) are bonded together.

3. A method for manufacturing disposable sanitary napkins (200) intended to be worn in an undergarment of a user, said method comprising:
(a) forming pairs of bridged flaps (10, 20) comprising the steps of
(i) providing a continuous web having a longitudinal axis and at least a garment-facing layer and an opposite layer;
(ii) bonding said superposed layers to form an area of juncture on said continuous web, said area of juncture defining a cyclic pattern and extending along said longitudinal axis;
(iii) severing said continuous web within said area of juncture to form first and second shaped strips (1, 2), each strip having:
(A) a first, substantially straight edge (101, 201);
(B) a second, shape-cut edge (102, 202) defining a plurality of projecting portions in a spaced apart relationship;
(C) at least one attachment portion disposed along the first edge between each of said projecting portions that unite said projecting portions to one another;
(iv) adjusting the two shaped strips (1, 2) to align the projecting portions of the first shaped strip (1) adjacent the projecting portions of the second shaped strip (2), whereby the adjusted shaped strips (1,2) have outer edges defined by the first, substantially straight edges (101, 201) of the first and second shaped strips (1, 2);
(v) applying zones of pressure sensitive adhesive (1111) to a continuous web of release liner (44), each zone of pressure sensitive adhesive (1111) corresponding to a projecting portion of the first and second shaped strips;
(vi) severing bridging segments of the release liner, each bridging segment having at least one zone of pressure sensitive adhesive arranged and configured for attachment to a projecting portion of the first shaped strip and at least one zone of pressure sensitive adhesive arranged and configured for attachment to a projecting portion of the second shaped strip, adjacent the projecting portion of the first shaped strip;
(b) forming a main body of a sanitary napkin, the main body:
(i) having a length, a width, and a thickness;
(ii) comprising an absorbent system, a topsheet, and a backsheet; and
(iii) having a pair of side edges extending along the length thereof;
(c) attaching the bridged flaps (10, 20) to the main body by bonding each shaped strip to the topsheet (21), wherein the first, substantially straight edge (101, 201) of each shaped strip is aligned with a corresponding side edge (29) of the main body which corresponds to a side edge of the topsheet (21), the bridged flaps are disposed over the topsheet (21), and each side flap (10, 20) is arranged and configured to be releasable from the bridging segment of the release liner and to be articulable away from the topsheet (21) of the sanitary napkin (200) and to be secured to a crotch portion of an undergarment for use; and
(d) enclosing the sanitary napkin (200) in a packaging material.

4. The method of claim 3 wherein the step of attaching the bridged flaps (10, 20) to the main body occurs as the main body is disposed on a support and travels in a direction perpendicular to its length transverse to the disposable sanitary napkin (200).

## Patentansprüche

1. Einweg-Damenbinde (200), die zum Tragen in der Unterwäsche einer Benutzerin beabsichtigt ist, wobei die Damenbinde (200) Folgendes umfasst:
(a) einen Hauptkörper,
(i) mit einer Länge, einer Breite und einer Dicke,
(ii) umfassend ein absorbierendes System (23), eine Deckschicht (21) und eine Bodenschicht (22) und
(iii) mit einem Paar Seitenränder, die sich entlang einer Länge davon erstrecken,
(b) ein Paar Brückenklappen (10, 20), umfassend
(i) einen ersten im Wesentlichen geraden Rand (101, 201),
(ii) einen zweiten zugeschnittenen Rand (102, 202), der einen vorragenden Abschnitt definiert,
(iii) ein Brückensegment eines Abziehstreifens mit mindestens einem Bereich aus Haftklebstoff, der zum Anbringen an dem vorragenden Abschnitt jeder Brückenklappe angeordnet und ausgestaltet ist,
wobei jede Brückenklappe (10, 20) so an der Deckschicht angebracht ist, dass der erste im Wesentlichen gerade Rand (101, 201) jedes zugeschnittenen Streifens auf einen entsprechenden Seitenrand (29) des Hauptkörpers ausgerichtet ist, um eine nach außen angeordnete Flanschdichtung zu bilden, die Brückenklappen (10, 20) über der Deckschicht (21) angeordnet sind und jede Seitenklappe so angeordnet und ausgestaltet ist, dass sie von dem Brückensegment des Abziehstreifens abgezogen und von der Deckschicht (21) der Damenbinde weggebogen werden kann und zum Gebrauch an einem Zwickelabschnitt der Unterwäsche befestigt werden kann, wobei die Flanschdichtungen nach innen zu dem Zwickelabschnitt der Unterwäsche hin angeordnet sind,
wobei jede Brückenklappe (10, 20) an die Deckschicht (21) gebondet ist und wobei der entsprechende Seitenrand des Hauptkörpers ein entsprechender Seitenrand der Deckschicht (21) ist.

2. Einweg-Damenbinde (200) nach Anspruch 1, wobei jede Brückenklappe (10, 20) in einem Umfangsbereich an die Deckschicht (21) gebondet ist, wobei die Deckschicht (21) und die Bodenschicht (22) aneinander gebondet sind.

3. Verfahren zur Herstellung von Einweg-Damenbinden (200), die zum Tragen in der Unterwäsche einer Benutzerin beabsichtigt sind, wobei das Verfahren Folgendes umfasst:
(a) Ausbilden von Paaren Brückenklappen (10, 20), umfassend die folgenden Schritte:
(i) Bereitstellen einer endlosen Bahn mit einer Längsachse und mindestens einer der Kleidung zugewandten Lage und einer gegenüberliegenden Lage,
(ii) Bonden der übereinanderliegenden Lagen zum Bilden eines Verbindungsstellenbereichs auf der endlosen Bahn, wobei der Verbindungsstellenbereich ein zyklisches Muster definiert und sich entlang der Längsachse erstreckt,
(iii) Trennen der endlosen Bahn in dem Verbindungsstellenbereich, um einen ersten und einen zweiten zugeschnittenen Streifen (1, 2) zu bilden, wobei jeder Streifen Folgendes aufweist:
(A) einen ersten im Wesentlichen geraden Rand (101, 201),
(B) einen zweiten zugeschnittenen Rand (102, 202), der eine Vielzahl von vorragenden Abschnitten mit einer beabstandeten Beziehung definiert,
(C) mindestens einen Anbringabschnitt, der entlang des ersten Rands zwischen jedem der vorragenden Abschnitte angeordnet ist, die die vorragenden Abschnitte miteinander vereinen,
(iv) Einstellen der beiden zugeschnittenen Streifen (1, 2), um die vorragenden Abschnitte des ersten zugeschnittenen Streifens (1) den vorragenden Abschnitten des zweiten zugeschnittenen Streifens (2) benachbart auszurichten, wodurch die eingestellten zugeschnittenen Streifen (1, 2) äußere Ränder haben, die von den ersten im Wesentlichen geraden Rändern (101, 201) des ersten und des zweiten zugeschnittenen Streifens (1, 2) definiert werden,
(v) Aufbringen von Haftklebstoffbereichen (1111) auf eine endlose Abziehstreifenbahn (44), wobei jeder Haftklebstoffbereich (1111) einem vorragenden Abschnitt des ersten und des zweiten zugeschnittenen Streifens entspricht,
(vi) Trennen von Brückensegmenten des Abziehstreifens, wobei jedes Brückensegment mindestens einen Haftklebstoffbereich, der zum Anbringen an einem vorragenden Abschnitt des ersten zugeschnittenen Streifens angebracht und ausgestaltet ist, und mindestens einen Haftklebstoffbereich, der zum Anbringen an einem vorragenden Abschnitt des zweiten zugeschnittenen Streifens neben dem vorragenden Abschnitt des ersten zugeschnittenen Streifens angebracht und ausgestaltet ist, hat,
(b) Ausbilden eines Hauptkörpers einer Damenbinde, wobei der Hauptkörper Folgendes aufweist:
(i) eine Länge, eine Breite und eine Dicke,
(ii) umfassend ein absorbierendes System, eine Deckschicht und eine Bodenschicht und
(iii) mit einem Paar Seitenränder, die sich entlang einer Länge davon erstrecken,
(c) Anbringen der Brückenklappen (10, 20) an dem Hauptkörper durch Bonden jedes zugeschnittenen Streifens an die Deckschicht (21), wobei der erste im Wesentlichen gerade Rand (101, 201) jedes zugeschnittenen Streifens auf einen entsprechenden Seitenrand (29) des Hauptkörpers ausgerichtet ist, der einem Seitenrand der Deckschicht (21) entspricht, die Brückenklappen über der Deckschicht (21) angeordnet sind und jede Seitenklappe (10, 20) so angeordnet und ausgestaltet ist, dass sie von dem Brückensegment des Abziehstreifens abgezogen und von der Deckschicht (21) der Damenbinde (200) weggebogen werden kann und zum Gebrauch an einem Zwickelabschnitt der Unterwäsche befestigt werden kann, und
(d) Umschließen der Damenbinde (200) in einem Verpackungsmaterial.

4. Verfahren nach Anspruch 3, wobei der Schritt des Anbringens der Brückenklappen (10, 20) an dem Hauptkörper erfolgt, wenn der Hauptkörper auf einem Träger angeordnet ist und sich in einer senkrecht zu seiner Länge verlaufenden Richtung quer zu der Einweg-Damenbinde (200) bewegt.

## Revendications

1. Serviette hygiénique jetable (200) destinée à être portée dans un sous-vêtement d'une utilisatrice, la serviette hygiénique (200) comprenant :
(a) un corps principal :
(i) ayant une longueur, une largeur et une épaisseur ;
(ii) comprenant un système absorbant (23), une feuille supérieure (21) et une feuille inférieure (22) ; et
(iii) ayant une paire de bords latéraux s'étendant sur la longueur associée ;
(b) une paire de rabats pontés (10, 20) comprenant
(i) un premier bord sensiblement droit (101, 201) ;
(ii) un second bord coupé en forme (102, 202) définissant une partie saillante ;
(iii) un segment de pontage d'une doublure antiadhésive comportant au moins une zone d'adhésif sensible à la pression conçue et configurée pour être fixée à la partie saillante de chaque rabat ponté ;
dans lequel chaque rabat ponté (10, 20) est fixé à la feuille supérieure de telle sorte que le premier bord sensiblement droit (101, 201) de chaque bande mise en forme soit aligné sur un bord latéral correspondant (29) du corps principal pour former un joint de bride disposé vers l'extérieur, les rabats pontés (10, 20) sont disposés sur la feuille supérieure (21), et chaque rabat latéral est conçu et configuré pour pouvoir être détaché du segment de pontage de la doublure antiadhésive et pour pouvoir être articulé à l'écart de la feuille supérieure (21) de la serviette hygiénique et pour être fixé à une partie d'entrejambe d'un sous-vêtement pour l'utilisation, moyennant quoi les joints de bride sont disposés vers l'intérieur en direction de la partie d'entrejambe du sous-vêtement,
dans lequel chaque rabat ponté (10, 20) est collé à la feuille supérieure (21), et dans lequel le bord latéral correspondant du corps principal est un bord latéral correspondant de la feuille supérieure (21).

2. Serviette hygiénique jetable (200) selon la revendication 1, dans laquelle chaque rabat ponté (10, 20) est collé à la feuille supérieure (21) dans une région périphérique dans laquelle la feuille supérieure (21) et la feuille arrière (22) sont collées ensemble.

3. Procédé de fabrication de serviettes hygiéniques jetables (200) destinées à être portées dans un sous-vêtement d'une utilisatrice, ledit procédé comprenant :
(a) la formation de paires de rabats pontés (10, 20) comprenant les étapes de
(i) fourniture d'une bande continue ayant un axe longitudinal et au moins une couche orientée vers le vêtement et une couche opposée ;
(ii) collage desdites couches superposées pour former une zone de jonction sur ladite bande continue, ladite zone de jonction définissant un motif cyclique et s'étendant le long dudit axe longitudinal ;
(iii) découpage de ladite bande continue à l'intérieur de ladite zone de jonction pour former des première et seconde bandes mises en forme (1, 2), chaque bande ayant :
(A) un premier bord sensiblement droit (101, 201) ;
(B) un second bord coupé en forme (102, 202) définissant une pluralité de parties saillantes dans une relation espacée ;
(C) au moins une partie d'attache disposée le long du premier bord entre chacune desdites parties saillantes qui unissent lesdites parties saillantes entre elles ;
(iv) ajustement des deux bandes mises en forme (1, 2) pour aligner les parties saillantes de la première bande mise en forme (1) adjacentes aux parties saillantes de la seconde bande mise en forme (2), moyennant quoi les bandes mises en forme ajustées (1, 2) ont des bords externes définis par les premiers bords sensiblement droits (101, 201) des première et seconde bandes mises en forme (1, 2) ;
(v) application de zones d'adhésif sensible à la pression (1111) sur une bande continue de doublure adhésive (44), chaque zone d'adhésif sensible à la pression (1111) correspondant à une partie saillante des première et seconde bandes mises en forme ;
(vi) découpe de segments de pontage de la doublure adhésive, chaque segment de pontage ayant au moins une zone d'adhésif sensible à la pression agencée et configurée pour être fixée à une partie saillante de la première bande mise en forme et au moins une zone d'adhésif sensible à la pression conçue et configurée pour être fixée à une partie saillante de la seconde bande mise en forme, adjacente à la partie saillante de la première bande mise en forme ;
(b) la formation du corps principal d'une serviette hygiénique, le corps principal :
(i) ayant une longueur, une largeur et une épaisseur ;
(ii) comprenant un système absorbant, une feuille supérieure et une feuille arrière ; et
(iii) ayant une paire de bords latéraux étendus sur la longueur associée ;
(c) la fixation des rabats pontés (10, 20) au corps principal en collant chaque bande mise en forme à la feuille supérieure (21), dans lequel le premier bord sensiblement droit (101, 201) de chaque bande mise en forme est aligné sur un bord latéral correspondant (29) du corps principal qui correspond à un bord latéral de la feuille supérieure (21), les rabats pontés sont disposés sur la feuille supérieure (21), et chaque rabat latéral (10, 20) est conçu et configuré pouvoir pour être détaché du segment de pontage de la doublure adhésive et pour pouvoir être articulé à l'écart de la feuille supérieure (21) de la serviette hygiénique (200) et pour être fixé à une partie d'entrejambe d'un sous-vêtement pour l'utilisation ; et
(d) l'enfermement de la serviette hygiénique (200) dans un matériau d'emballage.

4. Procédé selon la revendication 3 dans lequel l'étape de fixation des rabats pontés (10, 20) au corps principal se produit alors que le corps principal est disposé sur un support et se déplace dans une direction perpendiculaire à sa longueur transversale à la serviette hygiénique jetable (200).
